# EUROPÄISCHE PATENTSCHRIFT

(11) **EP 0 281 857 B1**
(45) Veröffentlichungstag und Bekanntmachung des Hinweises auf die Patenterteilung: **21.07.1993**
(21) Anmeldenummer: 88102782.5
(22) Anmeldetag: 25.02.1988
(51) Int. Cl.: A61F 13/15, A41B 13/04, B32B 7/12, C09J 5/00

(54) **Verfahren zum dauerhaften Verbinden von dehnbaren faden- oder bändchenförmigen Elementen auf einem flächigem Substrat sowie Anwendung desselben zur Herstellung von gerüschten Folienbahnabschnitten**
Method for securing elastic elements - strands or tapes - on a sheet, and use of this method in making elasticized portions of a web
Procédé de fixation d'éléments extensibles sous forme de fils de bandes sur un support en feuille et utilisation dudit procédé pour la fabrication d'éléments de feuille froncés

(30) Priorität: 07.03.1987 DE 3707349; 28.11.1987 DE 3740410
(43) Veröffentlichungstag der Anmeldung: 14.09.1988
(73) Patentinhaber: H.B. FULLER LICENSING & FINANCING, INC., Wilmington, Delaware 19801 (US)
(72) Erfinder: Werenicz, Harald, D-2120 Lüneburg (DE)
(74) Vertreter: UEXKÜLL & STOLBERG

(56) Entgegenhaltungen:
- DE-A- 3 016 197
- DE-A- 3 447 442
- DE-C- 3 347 294

## Beschreibung

Die Erfindung betrifft ein Verfahren zum dauerhaften Verbinden von faden- oder bändchenförmigen Elementen auf einem flächigen Substrat mittels eines Klebstoffes, wobei die faden- oder bändchenförmigen Elemente in gleichsinniger Richtung mit dem Substrat unterhalb von einen Schmelzklebstoff durchgehend oder intermittierend abgebenden Sprühköpfen transportiert werden, sowie die Anwendung dieses Verfahrens zur Herstellung von gerüschten Folienbahnabschnitten.

Es ist allgemein und insbesondere zur Herstellung von Windelhöschen z.B. aus DE-OS 34 47 442 und DE-PS 33 47 294 bekannt, beim Verbinden von faden- oder bändchenförmigen Elementen mit einem flächigen Substrat entweder die Oberflächen der beiden zu verbindenden Teile mit einem Klebstoff zu versehen und diese mit Klebstoff versehenen Oberflächen miteinander zu verpressen, oder nur die faden- oder bändchenförmigen Elemente bzw. nur das flächige Substrat mit einem Klebstoff zu versehen, worauf beide Teile miteinander verpreßt werden. Derartige Verfahren sind insbesondere bei der Herstellung von Massenartikeln sowohl zeitlich als auch apparativ aufwendig. Beispielsweise muß der Klebstoff mit Auftragwalzen oder Düsen mindestens auf das eine zu verklebende Element aufgebracht und dieses anschließend angepreßt werden. Die Verklebung von fadenförmigen Elementen auf dem flächigen Substrat birgt insbesondere dann Probleme, wenn es sich bei dem fadenförmigen Elementen um einen unter Spannung gehaltenen elastischen Faden handelt, wie er beispielsweise zum Anrüschen von dicht abschließenden flächigen Gebilden aus Kunststoff und/oder Wirrfaservlies benutzt wird, wie es im Hygienebereich z.B. für Windelhöschen, aber auch bei Arbeitsbekleidung erforderlich ist, um eine bessere Anschmiegbarkeit zu ermöglichen; ähnliche Probleme treten bei der Herstellung von OP-Hauben und Gesichtsmasken, die im chirurgischen Bereich verwendet werden, und bei zahlreichen anderen Artikeln im hygienischen und medizinischen Anwendungsbereich auf.

Man hat auch gemäß EP-A2 01 51 348 vorgeschlagen, gestreckte elastomere Bänder mit einer nicht elastischen thermoplastischen Folie zu verbinden und das Schichtgebilde dann unter Wärmeeinwirkung zu schrumpfen und dieses bei Wegwerfwindeln einzusetzen. Abgesehen von den Nachteilen bei der erforderlichen Wiedererwärmung zum Schrumpfen hat sich diese Arbeitsweise als unwirtschaftlich nicht durchgesetzt.

Aus der US-PS 43 79 016 ist es bekannt, nicht gespannte dehnbare bändchenförmige Elemente auf ein gerüschtes Substrat mittels eines Klebstoffes aufzubringen, wobei die nicht gespannten bändchenförmigen Elemente praktisch auf die vorstehenden Falten des gerüschten Substrats aufgeklebt werden.

Letztlich ist es aus der DE-PS 37 07 349 und der JP-OS 6 11 52 801 bekannt, zum dauerhaften Verbinden von dehnbaren faden- oder bändchenförmigen Elementen diese in gleichsinniger Richtung mit dem Substrat unterhalb von Sprühköpfen zu transportieren, die einen Schmelzklebstoff durchgehend oder intermittierend abgeben, wobei sich die faden- oder bändchenförmigen Elemente bereits in Kontakt mit dem Substrat befinden. Hierbei wird zwar eine gute Haftung erreicht, weil der Schmelzklebstoff sich auf das gedehnte faden- oder bändchenförmige Element und die benachbarten Bereiche des Substrats legt und das faden- oder bändchenförmige Element am Substrat hält. Es hat sich jedoch gezeigt, daß die Haftung des gedehnten Fadens am Substrat oder des ungedehnten Fadens auf einem gerüschten Substrat beim Auseinanderziehen desselben nicht ausreichend ist.

Die Erfindung hat sich die Aufgabe gestellt, ein neues Verfahren zum dauerhaften Verbinden von faden- oder bändchenförmigen Elementen auf einem flächigen Substrat mittels eines Klebstoffes vorzuschlagen, welches ohne großen Aufwand einsetzbar ist und zu einer dauerhaften Verbindung von insbesondere fadenförmigen Elementen auf einem flächigen Substrat geeignet ist, und zwar vorzugsweise bei unter Spannung stehenden elastischen Fäden, die mit einer Kunststoffunterlage verbunden werden sollen.

Die Erfindung hat sich ferner die Aufgabe gestellt, dieses neue Befestigungsverfahren für elastische Fäden bei der Herstellung von gewünschten Folienbahnabschnitten mit im jeweiligen Randbereich zwischen einer Folienbahn und einer Deckschicht befindlichen dehnbaren faden- oder bändchenförmigen Elementen, einer zwischen diesen angeordneten saugfähigen Einlage und einem diese abdeckenden Vliesabschnitt anzuwenden, bei dem die jeweiligen faden- oder bändchenförmigen Elemente unter Dehnspannng gehalten oder geführt und anschließend mit Schmelzklebstoff besprüht werden.

Zur Lösung der erfindungsgemäßen Aufgabe wird daher ein mal ein Verfahren gemäß Hauptanspruch vorgeschlagen, wobei besonders bevorzugte Ausführungsformen in den Unteransprüchen erwähnt sind, und zum anderen die Anwendung dieses Verfahrens gemäß Anspruch 12 und 13 vorgeschlagen.

Überraschenderweise hat sich gezeigt, daß man faden- oder bändchenförmige Elemente auf einem flächigen Substrat ganz einfach dadurch verkleben kann, daß man ohne vorherige Aufbringung von Klebstoff auf das flächige Substrat oder Beschichtung des faden- oder bändchenförmigen Elementes mit Klebstoff nunmehr das mit geringem Abstand zu dem flächigen Substrat angeordnete, fixierte bzw. geführte faden- oder bändchenförmigen Element mit einem versprühten Schmelzklebstoff besprüht, wobei der Schmelzklebstoff auch die Nachbarbereiche des faden- oder bändchenförmigen Elementes, also die angrenzenden Substratbereiche, erfassen soll.

Im Gegensatz zu konventionellen Klebeverfahren bedarf es nicht einer Anbringung einer Verklebungsschicht zwischen den beiden zu verklebenden Gegenständen, nämlich fadenförmigem Element und Substrat. Es genügt vielmehr die Ausbildung eines Sprühnebels des Schmelzklebstoffes, der sich beim Verfestigen als ein feines Gespinst von äußerst dünnen Fädchen des Schmelzklebstoffes sowohl um den Faden als auch in der Nachbarschaft desselben spinnenwebartig auf das Substrat legt und das faden- oder bändchenförmige Element fest an dem Substrat hält. Je nach Druck und Temperatur und je nach Art des Schmelzklebers können spinnwebartige Sprühfädchen bis zu musterartig sich überlappende Sprühfädchen in beispielsweise ringartiger, ovaler oder anderweitig mehr oder weniger gleichmäßig verschlungener Anordnung erzeugt und aufgebracht werden.

Überraschenderweise hat sich gezeigt, daß eine bessere Verklebung erzielt wird, wenn man das oder die faden- oder bändchenförmigen Elemente mit Abstand von 0,1 bis 3 cm zu dem flächigen Substrat in der gewünschten Lage anordnet und diese mit einem Schmelzklebstoff besprüht, wobei das oder die faden- oder bändchenförmigen Elemente in gleichsinniger Richtung mit dem Substrat unterhalb des Sprühkopfes transportiert und durch Verringerung des Abstandes zum Substrat in Kontakt gebracht werden.

Der Sprühnebel des Schmelzklebstoffes legt sich nicht nur als ein feines Gespinst von äußerst dünnen Fädchen des Schmelzklebstoffes um den oder die Fäden, sondern hängt von diesen herunter oder legt sich zum Teil auch über einen Nachbarfaden und gelangt durch den Luftstrom unter den oder die mit Abstand zur Substratbahn geführten Fäden und ergibt somit beim Kontakt zwischen Faden und Substrat einen spinnenwebartig auf dem Substrat quasi "eingesponnenes" Fadengebilde.

Für das erfindungsgemäße Verfahren können beliebige und an sich bekannte versprühbare Schmelzkleber eingesetzt werden, die einen Schmelzbereich von 80 bis 200°C haben und deren Viskosität es bei diesen Temperaturen ermöglicht, unter einem Druck von 1 bis 50 bar versprüht zu werden. Die Schmelzkleber haben im allgemeinen einen Erweichungspunkt von 70 bis 140°C und eine Viskosität bei 150°C in einem Bereich von etwa 500 bis 40 000 mPa.s. Der Verarbeitungsbereich dieser Schmelzkleber liegt im allgemeinen in einem Temperaturbereich von 105 bis 180°C.

Als Substrate werden im wesentlichen Kunststoffolien jeder Art beispielsweise solche auf Basis von Polyethylen oder Polypropylen verwendet, während als Fäden, die auf dem Substrat zu befestigen sind, übliche elastische Gummifäden oder Kunststoffäden, aber auch elastische bändchenförmige Elemente eingesetzt werden.

Bei beispielsweise der Herstellung von Windelhöschen, die im Schrittbereich gerüscht sein sollen, wird als Substrat eine Kunststoffolie und als Deckschicht eine Vliesbahn verwendet, zwischen denen das dehnbare oder gedehnte fädchen- oder bändchenförmige Element fixiert bzw. verklebt ist. Im allgemeinen wird der dehnbare Faden mit der Folie durch Aufbringen der spinnwebartigen Schmelzkleberfäden, meist durch Versprühen desselben, verklebt und unmittelbar darauf wird die Vliesschicht aufgedrückt, die sich mit dem noch klebenden Schmelzkleber haftend verbindet. Analog kann der dehnbare Faden auch erst mit der Vliesbahn verbunden werden, die dann mit der Folie verpreßt wird.

Wenngleich die Erzeugung der spinnwebartigen Schmelzkleberlage durch Versprühen auf den mit Abstand zu dem Substrat geführten dehnbaren Faden erfolgt, ist es auch möglich, das Schmelzklebergespinst gesondert herzustellen und dann an die Substrat bahn heranzuführen.

Im allgemeinen wird das faden- oder bändchenförmige Element in gedehntem Zustand mit Abstand zu dem Substrat geführt. Nach dem Verkleben und Schneiden zieht sich dieser Bereich zusammen und ergibt die gewünschte Rüschung. In dem nicht zu rüschenden Bereich erfolgt keine Verklebung und der lose auf dem Substrat noch unter Dehnspannung stehende Faden wird beim Zuschneiden der Folienabschnitte zertrennt.

Bei einer anderen Ausführungsform des erfindungsgemäßen Verfahrens kann man den Faden in nicht oder nur geringfügig gestrecktem Zustand mit Abstand zu dem bereits gefälteten Substrat führen und verkleben. Dieses Verfahren hat den Vorteil, daß die nicht für den gerüschten Bereich gedachten dehnbaren Fäden unmittelbar hinter der Fältung oder Rüschung abgeschnitten werden können. Auch dieses Verfahren läßt sich kontinuierlich beispielsweise bei der Herstellung von Windelhöschen unter Verwendung einer die zu rüschenden Bereiche aufnehmenden Walze mit entsprechenden Vertiefungen gemäß EU-A1-0 154 068 durchführen.

Nach einem weiteren Aspekt der vorliegenden Erfindung kann das erfindungsgemäße Verfahren auch zur Herstellung von gerüschten Folienbahnabschnitten mit im jeweiligen Randbereich zwischen einer Folienbahn und einer Deckschicht befindlichen dehnbaren faden- oder bändchenförmigen Elementen und einer zwischen diesen angeordneten saugfähigen Einlage und einer Abdeckschicht angewendet werden.

Bei der Herstellung von Höschenwindeln ist nicht nur der gerüschte oder gekräuselte Abschnitt im späteren Schrittbereich der Windel und die Abdeckung mit einer Vliesschicht erforderlich; es muß auch noch eine im Mittelbereich der Folie liegende saugfähige Einlage vorgesehen werden. Bei der Herstellung derartiger Windeln ist es zweckmäßig, die saugfähige Einlage im Schrittbereich mit der Folie oder mit einer auf der Folie aufliegenden Zwischenschicht zu verkleben. Zur Vereinfachung des Herstellungsverfahrens wird daher das erfindungsgemäße Verfahren angewandt, wie es in den Unteransprüchen 12 und 13 aufgeführt ist.

Hierbei wird das Aufbringen der spinnwebartigen Schmelzfäden auf den dehnbaren Faden noch auf den Zwischenbereich zwischen den jedweiligen am Bahnrand angeordneten dehnbaren Fäden ausgedehnt. Man läßt also bei den betreffenden Bereichen den Sprühnebel aus Schmelzklebstoff sich nicht nur auf den dehnbaren Faden und dessen benachbarten Bereich ablagern, sondern besprüht auch den zwischen den dehnbaren Fäden liegenden Bereich, damit sich die später eingeführte Bahn aus saugfähigem Material mit der Polyethylenfolie verhaftet.

Aus optischen und sonstigen Gründen ist es in vielen Fällen zweckmäßig, diesen Zwischenbereich auf der Polyethylenfolie nicht direkt zu besprühen, sondern eine in diesem Mittelbereich geführte dünne Schicht aus vliesartigem Material mit Klebstoff zu versehen. Dieses hat den Vorteil, daß etwaige Verklebungsstellen, die sonst auf der Polyethylenfolien-Innenfläche wären und von außen erkennbar sind, nicht in Erscheinung treten. Bei dieser Aufbringung von spinnwebartigen Schmelzkleberfädchen in dem zwischen den dehnbaren Fäden liegenden Zwischenbereich kann die Klebstoffverteilung so vorgenommen werden, daß die im Zwischenbereich besprühte Fläche kürzer ist als die in den Randbereichen.

Im folgenden soll die Erfindung anhand von Zeichnungen näher erläutert werden; es zeigen:
- Figur 1: eine teilweise geschnittene Darstellung eines dehnbaren Fadens, der auf einem Substrat nach dem erfindungsgemäßen Verfahren befestigt ist,
- Figur 2: eine schematische Darstellung, wie mit Abstand zu einem Substrat ein gedehntes Bandgebilde besprüht und mit ersterem zusammengeführt und mit einer Deckschicht abgedeckt wird,
- Figur 3: eine Ansicht analog Figur 2,
- Figur 4: zwei schematische Darstellungen von spinnwebartigen bzw. ornamentartigen Schmelzkleberfädchen.
- Figur 5: eine teilweise geschnittene Darstellung der Aufbringung eines nicht oder nur gering gedehnten fadenförmigen Elementes auf einer vorgefalteten Bahn,
- Figur 6: eine schematische Schnittdarstellung der Aufbringung eines gering gedehnten Fadens mit einem kontinuierlichen Fältelungsrad,
- Figur 7: eine schematische Darstellung der Anwendung des erfindungsgemäßen Verfahrens bei der kontinuierlichen Herstellung von Windelhöschen,

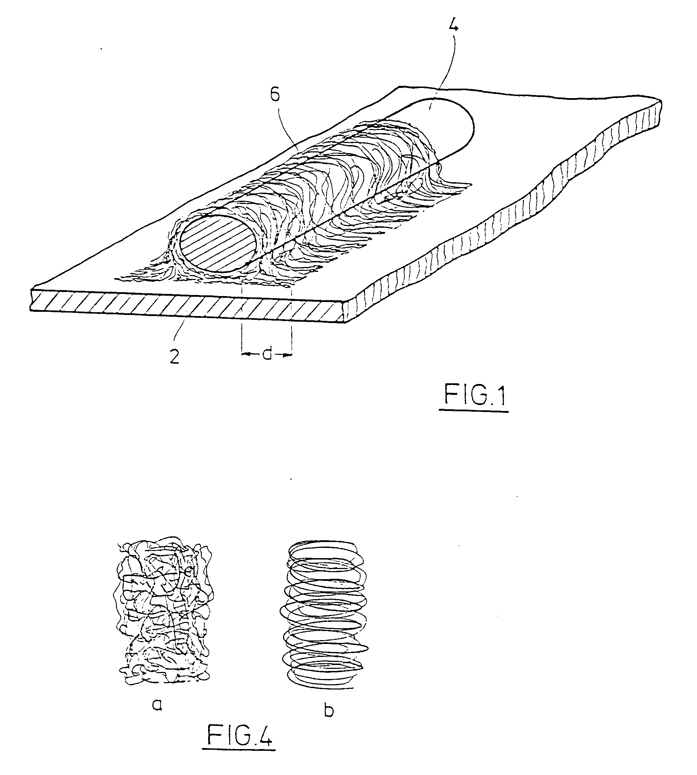

Figur 1 zeigt einen Abschnitt eines Substrates 2, das im vorliegenden Fall eine Polyethylenfolie mit einer Wandstärke von 50 µm ist. Auf dieser ist ein Faden 4 aus dehnbarem Kunststoff aufgebracht und mit einer Vielzahl von spinnwebartig aufeinanderliegenden Schmelzkleberfädchen gehalten. Da der Faden während des Besprühens mit Klebstoff mit Abstand zum Substrat geführt und dann mit diesem in Kontakt gebracht worden ist, sind die spinnwebartigen Schmelzkleberfädchen zum Teil unter das vorher mit Abstand zur Folie geführte Fadengebilde bewegt oder gedrängt worden; die restlichen bedecken die erreichbare Oberfläche des Fadens 4 und liegen noch auf dem angrenzenden Bereich d des Substrates 2.

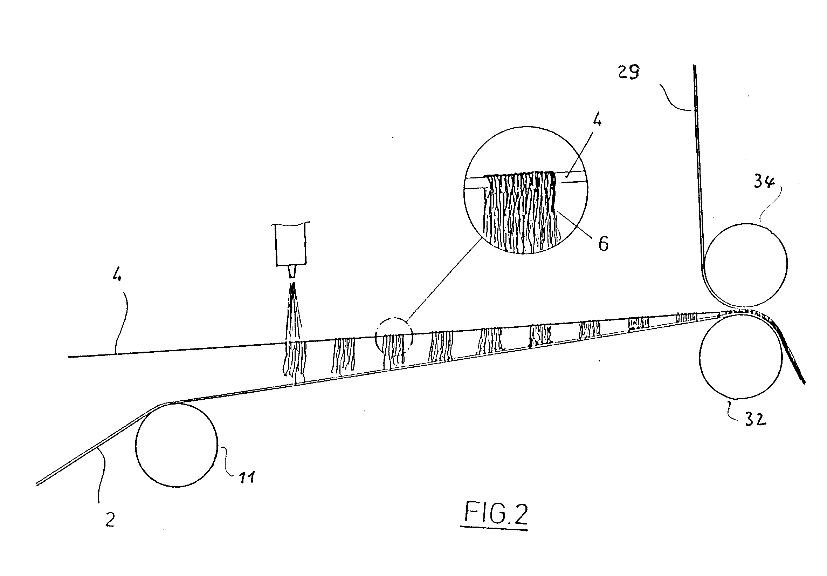

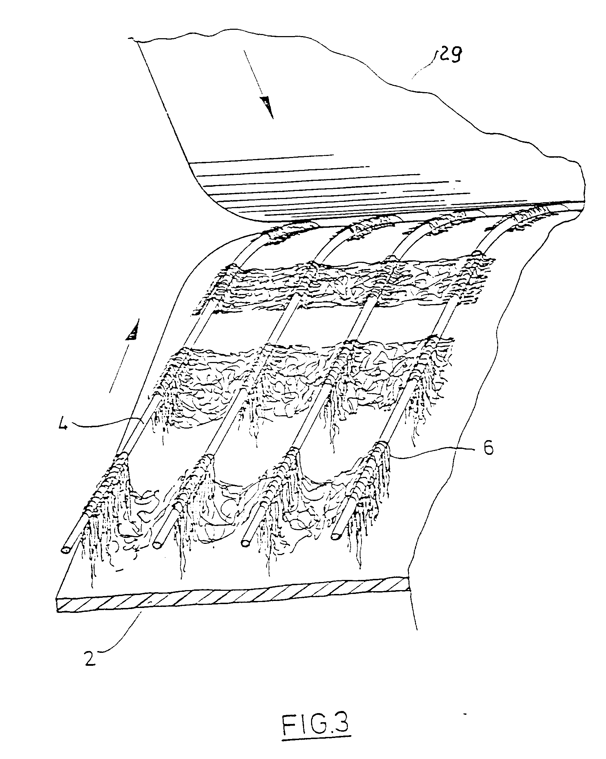

Bei den in Figur 2 und Figur 3 gezeigten Ausführungsformen ist erkennbar, wie die elastischen Bändchen 4 mit Abstand zu dem Substrat, welches in Figur 2 eine Polyethylenfolie 2 und in Figur 3 ein Polypropylen-Wirrfaservlies 2 ist, geführt werden, wobei spinnwebartige Schmelzkleberfädchen 6 herabhängen und sich beim Zusammenführen mit dem Substrat verhaften und mit einer Deckschicht, und zwar einem Polypropylenvlies 29 abgedeckt werden.

Figur 5 zeigt, wie eine vorgefaltete Substratbahn 12 an ihrem Scheitelpunkt 13 von einem in diesem Fall nur gering oder gar nicht gespanntem elastischen Band 14 belegt ist und mit den Schmelzkleberfädchen 16 bedeckt sind.

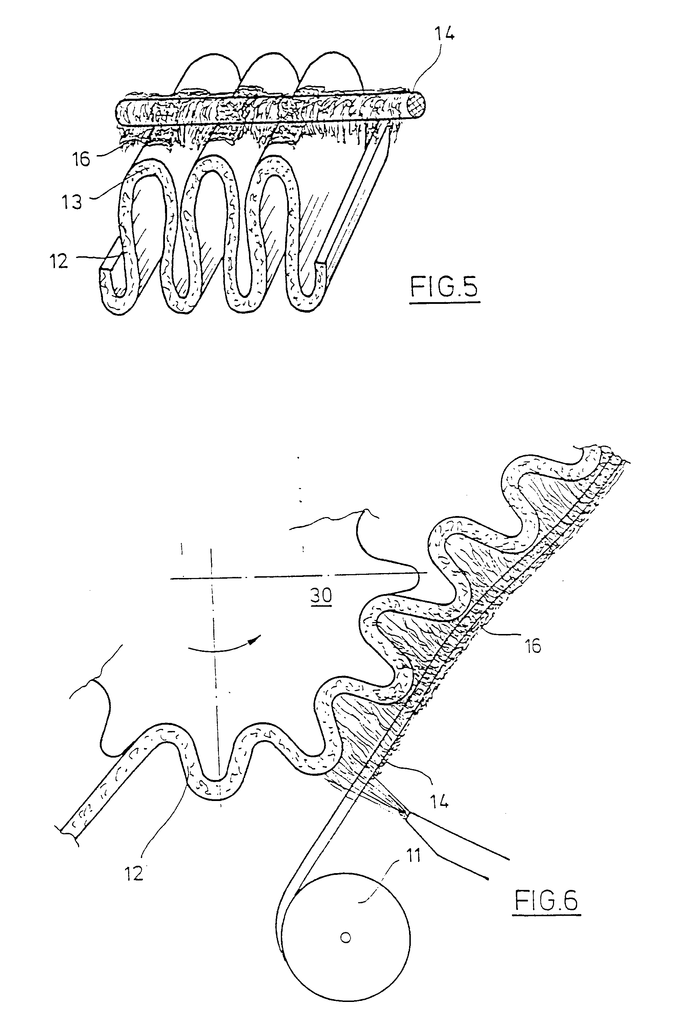

Analog wird bei der in Figur 6 gezeigten schematischen Darstellung mittels eines Rüschenrades oder Rüschenwalze 30 mit am Umfang angeordneten Vertiefungen das Substrat 12 in eine gefaltete Konfiguration gebracht, wobei wiederum an den Scheitelpunkten ein dehnbarer Faden 14 von einer Rolle 11 tangential belegt und mittels Düsen mit Kunststoffädchen 16' spinnwebartig verklebt und zur Weiterverarbeitung in Laufrichtung abgezogen wird.

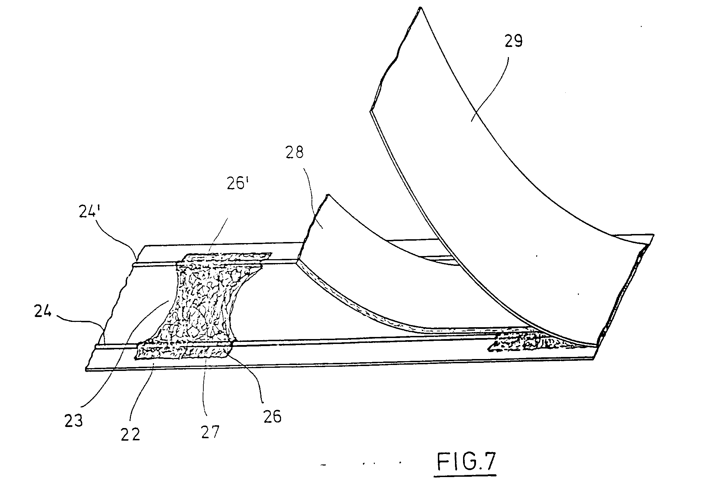

Bei der in Figur 7 schematisch dargestellten Anwendung des erfindungsgemäßen Verfahrens bei der Herstellung von einer für Höschenwindeln geeigneten Folienbahn wird die Folienbahn 22 als Substrat einer ersten Station zugeführt, in welcher zwei elastische oder dehnbare Fäden 24 bzw. 24' im Randbereich mit Abstand zur Folienbahn geführt und mit Schmelzklebstoff in den Bereichen 26 und 26' besprüht worden sind. Der Klebstoff reicht von der Seitenkante des Fadens mit einem Abstand von 1 bis 15 mm über den Faden hinaus Jedoch ist im vorliegenden Fall auch der Mittelbereich 27 des Substrates besprüht, wobei die quer zur Bahnrichtung liegenden Begrenzungslinien des besprühten Bereiches 27 zur Mittelachse hin konkav eingezogen sind, wie es bei 23 gezeigt ist.

In einer zweiten Station wird eine saugfähige Einlage 28 als Bahn in den Mittelbereich zwischen den elastischen Fäden 24, 24' eingebracht, diese wird in einer dritten Station mit einer Vliesbahn 29 bis über den Bereich der mit Schmelzklebstoff bedeckten dehnbaren Fäden 24 und 24' abgedeckt und anschließend verpreßt. Am Ende dieser Station werden die einzelnen Folienabschnitte mit einem Schneidwerkzeug in Abschnitte 31 geschnitten.

Bei der eingangs erwähnten Verfahrensvariante mit geringer Abstandsführung der elastischen Bänder 4 gegenüber dem Substrat 2, werden, wie in Figur 2 und Figur 3 gezeigt, die elastischen Bändchen 4 mit Abstand zu dem Substrat, das beispielsweise eine Polyethylenfolie 2 ist, geführt, wobei die über eine Düse 8 aufgesprühten Schmelzkleberfäden 6 herabhängen und sich beim Zusammenführen mit der Substratbahn bei Verringerung des Abstandes von z.B. anfänglich etwa 1 bis 2 cm bis zum Kontakt mit der Folienbahn 2 verteilen und auch unter die Fadengebilde gelangen und letzlich mit den spinnwebartigen Schmelzkleberfädchen 6 auf dem Substrat 2 verhaftet und mit einer Deckschicht, und zwar einen Polypropylenvlies 29 abgedeckt werden. Die Folienbahn 2 wird über eine Walze 11 der Klebersprühstation zugeführt und über die Walze 32 abgeführt. Oberhalb dieser Walze 32 befindet sich eine die Vliesbahn 29 zuführende Walze 34. In dem Spalt zwischen den Walzen 32 und 34 oder kurz vorher findet der Kontakt der mit Klebstoff-Fäden 6 belegten Bändchen 4 mit dem Substrat 2 statt, wobei die herunterhängende Klebstoffädchen 6 schon vorher mit dem unter einem spitzen Winkel von etwa 5 bis 10° zu den Bändchen 4 zugeführten Substrat 2 in Berührung gelangen und ein spinnwebartiges "Bett" oder eine Unterlage aus Klebstoff-Fädchen für das Bändchen 4 bilden. Im Spaltbereich der Walzen 32 und 34 erfolgt dann auch die Verbindung der zugeführten Vliesbahn 29 mit dem Substrat 2, wobei die Haftung dieser durch den in der Breite (d) beiderseits des Bändchens 4 aufgesprühten Schmelzklebstoff erfolgt. Auf ähnliche Weise kann auch vorher oder statt der Vliesbahn eine saugfähige Einlage 28 - wie in Figur 7 gezeigt - aufgebracht werden, wonach gegebenenfalls die Vliesbahn in einer weiteren Station dem Substrat zugeführt und mit diesem verbunden wird.

Bei der Verfahrensvariante mit geringfügiger Abstandsführung des elastischen Bändchens 4 gegenüber dem Substrat 2 und der Abwandlung ein nicht oder nur gering gespanntes elastisches Bändchen 4 auf einem bereits vorgefältetem Substrat 12 bzw. 12' analog Figur 5 oder Figur 6 zu befestigen, wird - wie in Figur 6 gezeigt - die Versprühung des Schmelzklebers in Laufrichtung der Substratbahn 12 und des elastischen Bändchens 14 etwas zurückverlegt, also in Richtung auf die Zuführwalze 11, die das oder die elastischen Bändchen 14 dem in dem Fälterungsrad 30 transportiertes Substrat 12 zuführt. Der Klebstoff wird bei dieser Verfahrensvariante auf das elastische Bändchen 14 aufgesprüht, bevor dieses mit dem hochgefalteten Substrat in dessen Scheitelpunkt in Kontakt gelangt. Hierbei bildet sich unterhalb des elastischen Bändchens analog Figur 8 bis 10 ein "Bett" aus einem Klebfädchengespinst aus, auf dem das elastische Bändchen aufliegt und ferner spinnwebartig von oben durch das Klebfädchengespinst an bzw. mit dem Substrat verhaftet wird.

Im folgenden soll die Erfindung anhand von Beispielen näher erläutert werden.

### Beispiel 1

Um bei einem hygienischen Artikel ein Substrat aus einer Polyethylenfolie mit einer Foliendicke von 20 bis 25 µm in einen gerüschten Zustand zu bringen, wurde mit 10 mm Abstand zu dieser Folienbahn ein gedehnter elastischer Faden aus einem Polyether-Copolymeren (z.B. Lycra® XA) mit einer Fadenstärke von 940 dtex geführt und mit einem Schmelzkleber bestehend aus 50% Kohlenwasserstoffharz mit einem R+K-Erweichungspunkt von 115°C, 30% eines Styrol-Butadien-Styrol-Blockcopolymeren und 20% Mineralöl, bei einer Temperatur von 175°C bei Drücken von 2 bis 8 bar in einem Breitenbereich von 15 mm intermittierend in Abschnitten von 25 cm Länge besprüht, wobei die Auftragstärke 25 g/m² betrug Anschließend wurde die Folienbahn mit dem Fadengebilde in Kontakt geführt und in den nicht besprühten Bereichen quer abgeschnitten, wonach die gewünschten gerüschten Bahnteile erhalten wurden.

### Beispiel 2

Bei einem weiteren Versuch wurde analog Beispiel 1 gearbeitet, wobei anschließend auf den vorher befestigten Faden ein Polypropylen-Spinnvlies mit einer Dicke von etwa 0,22 mm und einem Flächengewicht von etwa 20 g/m² aufgepreßt wurde, so daß der gesamte Bereich der Folienbahn mit einem angemessenen Abstand von Rand der Folienbahn mit diesem Spinnvlies bedeckt war, welches auf dem mit Schmelzkleber beschichteten fadenförmigen Band haftete.

### Beispiel 3

Es wurde ein dehnbares Band aus Polyether-Copolymeren mit einer Breite von 3 mm und einer Dicke von 0,3 mm mit Abstand von 15 mm zu einem thermisch gebundenen Polypropylenstapelfaservlies geführt und in einem Breitenbereich von 10 mm mit dem Schmelzkleber gemäß Beispiel 1 besprüht, wobei die Auftragsstärke 42 g/m² betrug. Nach Verringerung des Abstandes erfolgte die Verklebung, wonach eine Polypropylenfolie aufgepreßt und die erhaltene Bahn in Abschnitte weiter verarbeitet wurde.

### Beispiel 4

Es wurde analog Figur 6 auf einer kontinuierlich arbeitenden Anlage zur Herstellung von Windelhöschen eine Polyethylenfolie mit einer Breite von 22 cm zugeführt, die mit der in Figur 6 gezeigten Rüschenwalze abschnittsweise durch Vakuum mit etwa 7 mm tiefen Fältelungen in einem Abstand von 3 mm versehen wurde. In Bahnrichtung wurden unter geringer Spannung jeweils im Randbereich der Folie ein dehnbares Band aus Polyurethan mitgeführt, welches intermittierend mit Klebstoff besprüht und dann von der Rüschenwalze abgezogen und weiterbearbeitet wurde.

### Beispiel 5

Es wurde wie in Figur 7 gezeigt, eine glatte Folienbahn aus Polyethylen auf einer Windelanlage verarbeitet; auf die Folienbahn wurden zwei dehnbare Bänder aus Polyurethan analog Beispiel 1 unter Dehnspannung mit Abstand zur Folie geführt und mit dem Schmelzklebstoff gemäß Beispiel 1 besprüht. Der spinnwebartig sich ablegende Schmelzkleber hatte von den dehnbaren Bändern jeweils nach außen einen Abstand von 10 mm, erstreckte sich aber auch in den Mittelbereich 27 zwischen den beiden Bändern, wobei der Schmelzkleberauftrag mit 3 Sprühköpfen durchgeführt wurde, von denen die beiden äußeren für den Auftrag auf die dehnbaren Bänder entsprechend schmal und lang, und der für den Mittelbereich breiter und kürzer waren. Nach Kontakt von Substrat und den dehnbaren Bändern wurde in den Bereich zwischen den Bändern eine saugfähige Faserbahn mit einer Dicke von etwa 12 mm eingeführt und anschließend mit der in Beispiel 3 verwendeten Polypropylen-Spinnvliesbahn 29 abgedeckt. Am Ende der Station wurde die fertige Bahn durch ein Schneidwerkzeug quer zur Bahnrichtung zu einzelne Abschnitte durchtrennt.

### Beispiel 6

Bei einer Abwandlung des Verfahrens gemäß Beispiel 5 wurde mit der zugeführten Folienbahn im Bereich zwischen den gespannten Fäden eine dünne Faservliesbahn eingelegt, wobei bei dem nachträglichen Aufbringen des Klebstoffes auf die Fäden und auf den Mittelbereich der Schmelzklebstoff im Mittelbereich 27 nicht mit der Polyethylenfolie in Berührung kam und nur die saugfähige Einlage im Schrittbereich mit verklebte. Da diese zwischengelegte Vliesschicht zuvor auf der Polyethylenfolie fixiert wurde, kann die saugfähige Einlage im Rüschenbereich nicht verrutschen, zumal sie nachträglich noch von der Deckschicht abgedeckt wird, die ebenfalls in den Randbereichen mit der Kunststoffolie verklebt wird.

In allen Fällen wird eine ausgezeichnete Haltbarkeit der Verklebung insbesondere eine gute Creep-Beständigkeit erreicht; ferner kann mit dem erfindungsgemäßen Verfahren eine bessere Rüschung erreicht werden, weil die Klebstoffäden im Vergleich zu einer Klebstoffspur der Kontraktion der elastischen Fäden einen geringeren Widerstand entgegensetzen, was zu einem sehr viel schmiegsameren Anliegen von beispielsweise Windeln führt.

## Patentansprüche

1. Verfahren zum dauerhaften Verbinden von ein oder mehreren dehnbaren faden- oder bändchenförmigen Elementen auf einem flächigen Substrat mittels eines Klebstoffes wobei die faden- oder bändchenförmigen Elemente in gleichsinniger Richtung mit dem Substrat unterhalb von einen Schmelzklebstoff durchgehend oder intermittierend abgebenden Sprühköpfen transportiert werden, **dadurch gekennzeichnet,** daß die faden- oder bändchenförmige Elemente (4) mit Abstand zu dem flächigen Substrat (2) in der gewünschten Lage angeordnet werden und nach dem Besprühen mit Schmelzklebstoff durch Verringerung des Abstandes zum Substrat in Kontakt mit dem Substrat gebracht werden.

2. Verfahren nach Anspruch 1, **dadurch gekennzeichnet,** daß die faden- oder bändchenförmigen Elemente (4) während des Besprühens und beim Kontakt mit dem Substrat unter Dehnspannung gehalten werden.

3. Verfahren nach Anspruch 2, **dadurch gekennzeichnet,** daß die unter Spannung stehenden faden- oder bändchenförmigen Elemente (4), nachdem sie mit einem Abstand von 0,5 bis 3 cm von dem Substrat mit dem Schmelzklebstoff besprüht worden sind, unter einem spitzen Winkel mit dem Substrat zusammengeführt werden; (Fig.2).

4. Verfahren nach Anspruch 1, **dadurch gekennzeichnet,** daß zumindest die vorstehenden Bereiche (13) eines gerüschten oder gefalteten Substrates (12) und die nicht oder nur geringfügig unter Spannung stehende faden- oder bändchenförmige Elemente (14), die mit Abstand zu dem Substrat (12) angeordnet oder geführt sind, nach dem Besprühen mit dem Schmelzklebstoff (16) mit dem Substrat in Kontakt gebracht werden; (Fig. 6).

5. Verfahren nach einem der Ansprüche 1 bis 4, **dadurch gekennzeichnet,** daß der Schmelzklebstoff in Form von spinnwebartig bis musterartig sich überlappenden Sprühfädchen mit einem Durchmesser von 20 bis 400 µm auf das oder die faden- oder bändchenförmigen Elemente (4, 14) aufgesprüht wird.

6. Verfahren nach einem der Ansprüche 1 bis 5, **dadurch gekennzeichnet,** daß die faden- oder bändchenförmige Elemente (4, 14) und die unterhalb dieser liegenden Bereiche des Substrates in einer Breite (d) von 1 bis 15 mm, gemessen von der Längskante des nachher mit dem Substrat verbundenen jeweiligen faden- oder bändchenförmigen Elementes, mit dem versprühten Klebstoff abgedeckt werden.

7. Verfahren nach einem der Ansprüche 1 bis 6, **dadurch gekennzeichnet,** daß der Schmelzklebstoff in einer Menge von 2 bis 100 g/m² auf die faden- oder bändchenförmigen Elemente (4, 14) aufgebracht wird.

8. Verfahren nach einem der Ansprüche 1 bis 7, **dadurch gekennzeichnet,** daß als Substrat (2) eine Kunststoffolie oder eine imprägnierte Papier- bzw. Vliesbahn verwendet wird.

9. Verfahren nach einem der Ansprüche 1 bis 8, **dadurch gekennzeichnet,** daß auf das Substrat und auf die faden- oder bändchenförmigen Elemente nach dem Aufsprühen des Schmelzklebstoffes und unmittelbar nach dem Zusammenführen dieser mit dem Substrat eine Deckschicht aufgebracht und danach gegebenenfalls angepreßt.

10. Verfahren nach einem der Ansprüche 1 bis 9, **dadurch gekennzeichnet,** daß als Substrat eine Folie (2) und als Deckschicht ein Vlies (8) verwendet wird.

11. Verfahren nach einem der Ansprüche 1 bis 9, **dadurch gekennzeichnet,** daß als Substrat ein Vlies und als Deckschicht eine Folie verwendet wird.

12. Verfahren nach den Ansprüchen 1 bis 11, **dadurch gekennzeichnet,** daß das Verfahren angewandt wird zur Herstellung von gerüschten Folienbahnabschnitten mit im jeweiligen Randbereich zwischen einer Folienbahn und einer Deckschicht befindlichen dehnbaren faden- oder bändchenförmigen Elementen, einer zwischen diesen angeordneten saugfähigen Einlage und einem diese abdeckenden Vliesabschnitt, bei dem die jeweiligen faden- oder bändchenförmigen Elemente unter Dehnspannung gehalten oder geführt und anschließend intermittierend mit dem Schmelzklebstoff besprüht werden, wobei
a) die faden- oder bändchenförmigen Elemente sowie deren angrenzende Bereiche und gleichzeitig der zwischen den faden- oder bändchenförmigen Elementen liegende Bereich mit Schmelzklebstoff besprüht und die bändchenförmigen Elemente mit dem Substrat durch Zusammenführen in Kontakt gebracht werden, und danach
b) auf den zwischen dem faden- oder bändchenförmigen Element mit Schmelzklebstoff besprühten Bereich eine Bahn aus saugfähigem Polstermaterial aufgebracht wird,
c) auf die Folienbahn und die Bahn aus saugfähigem Polstermaterial eine Vliesbahn derart aufgebracht wird, daß diese sowohl das saugfähige Polstermaterial bedeckt als auch im Bereich der mit Schmelzklebstoff besprühten faden- oder bändchenförmigen Elemente aufliegt,
d) die Vliesbahn mindestens im Bereich der faden- oder bändchenförmigen Elemente mit diesen verpreßt wird und
e) das derart erhaltene Verbundmaterial quer zur Bahnrichtung in Abschnitte getrennt wird.

13. Verfahren nach Anspruch 12, **dadurch gekennzeichnet,** daß die mit Schmelzklebstoff besprühten Bereiche der in einem Abstand zur Folienbahn von 3 bis 0,5 cm geführten faden- oder bändchenförmigen Elemente unter einem spitzen Winkel mit der Folienbahn zusammengeführt und anschließend die Bahn aus saugfähigem Polstermaterial und die Vliesbahn aufgebracht werden.

## Claims

1. Method for permanently bonding one or a plurality of elastic strand-shaped or tape-shaped elements on a flat substrate by means of an adhesive, wherein the strand-shaped or tape-shaped elements are transported in the same direction with the substrate below spray heads emitting a hot-melt-type adhesive continuously or intermittently, characterised in that the strand-shaped or tape-shaped elements (4) are disposed in the desired position at a distance to the flat substrate (2) and, after having been sprayed with the hot-melt-type adhesive they are brought into contact with the substrate by reducing the distance to the substrate.

2. Method according to claim 1, characterised in that the strand-shaped or tape-shaped elements (4) are held in an elastically tensioned manner during the spraying process and when in contact with the substrate.

3. Method according to claim 2, characterised in that the tensioned strand-shaped or tape-shaped elements (4) are brought together with the substrate at an acute angle, after they have been sprayed with a hot-melt-type adhesive at a distance of 0.5 to 3 cm from the substrate; (Fig. 2).

4. Method according to claim 1, characterised in that at least the upwardly protruding regions (13) of a ruched or folded substrate (12) and the strand-shaped or tape-shaped elements (14) which are not tensioned or which are only slightly tensioned and which are disposed or guided at a distance to the substrate (12), are brought into contact with the substrate after having been sprayed with a hot-melt-type adhesive (16); (Fig. 6).

5. Method according to one of claims 1 to 4, characterised in that the hot-melt-type adhesive is sprayed onto the strand-shaped or tape-shaped element(s) (4, 14) in the form of small spray strands which have a diameter of 20 to 400 µm and which overlap each other in the form of a spider's web or a pattern.

6. Method according to one of claims 1 to 5, characterised in that the strand-shaped or tape-shaped elements (4, 14) and the regions of the substrate lying below these elements (4, 14) are covered with the sprayed-on adhesive over a width (d) of 1 to 15 mm, measured from the longitudinal edge of each strand-shaped or tape-shaped element which is subsequently bonded to the substrate.

7. Method according to one of claims 1 to 6, characterised in that the hot-melt-type adhesive is applied to the strand-shaped or tape-shaped elements (4, 14) in a quantity of 2 to 100 g/m².

8. Method according to one of claims 1 to 7, characterised in that a synthetic material film or an impregnated paper run and/or fleece run is used as the substrate (2).

9. Method according to one of claims 1 to 8, characterised in that a cover layer is applied to the substrate and to the strand-shaped or tape-shaped elements after the hot-melt-type adhesive has been sprayed on and immediately after the elements have been brought together with the substrate and the cover layer is then, if necessary, pressed on.

10. Method according to one of claims 1 to 9, characterised in that a film (2) is used as the substrate and a fleece (8) is used as the cover layer.

11. Method according to one of claims 1 to 9, characterised in that a fleece is used as the substrate and a film is used as the cover layer.

12. Method according to claims 1 to 11, characterised in that the method is employed for the purpose of producing portions of ruched film runs having in each edge region elastic strand-shaped or tape-shaped elements located between a film run and a cover layer, furthermore having an absorbent insert disposed between these elements and a portion of fleece covering this insert, wherein the respective strand-shaped or tape-shaped elements are held or guided under elastic tension and are subsequently intermittently sprayed with the hot-melt-type adhesive, wherein
a) the strand-shaped or tape-shaped elements as well as the regions adjacent thereto and at the same time the region lying between the strand-shaped or tape-shaped elements is sprayed with hot-melt-type adhesive and the tape-shaped elements are brought together into contact with the substrate, and subsequently
b) a run of absorbent bolstering material is applied to the region which has been sprayed with hot-melt-type adhesive and which lies between the strand-shaped or tape-shaped element,
c) a fleece run is attached to the film run and the run of absorbent bolstering material in such a way, that the fleece run covers both the absorbent bolstering material and also lies in the region of the strand-shaped or tape-shaped elements which have been sprayed with hot-melt-type adhesive,
d) the fleece run is pressed together with the film run and the run of absorbent bolstering material at least in the region of the strand-shaped or tape-shaped elements and
e) the composite material obtained in this way is separate in parts transverse to the direction of the run.

13. Method according to claim 12, characterised in that the regions, which have been sprayed with hot-melt-type adhesive, of the strand-shaped or tape-shaped elements, which are guided at a distance of 3 to 0.5 cm to the film run, are brought together with the film run at an acute angle and the run of absorbent bolstering material and the fleece run are subsequently applied.

## Revendications

1. Procédé de fixation durable d'un ou plusieurs éléments extensibles en forme de fils ou de bandelettes sur un support mince au moyen d'un adhésif, les éléments en forme de fils ou de bandelettes défilant dans le même sens que le support sous des têtes de pulvérisation projetant, de manière continue ou intermittente, un adhésif fusible, caractérisé en ce que les éléments (4) en forme de fils ou de bandelettes se trouvent, dans la position souhaitée, à distance du support mince (2) et, après pulvérisation avec de l'adhésif fusible, sont mis en contact avec le support par la diminution de la distance par rapport au support.

2. Procédé selon la revendication 1, caractérisé en ce que les éléments (4) en forme de fils ou de bandelettes sont maintenus sous une tension d'allongement pendant la pulvérisation et lors du contact avec le support.

3. Procédé selon la revendication 2, caractérisé en ce que, après avoir été pulvérisés avec l'adhésif fusible à une distance de 0,5 à 3 cm du support, les éléments (4) en forme de fils ou de bandelettes soumis à une tension sont guidés conjointement avec un angle aigu par rapport au support ; (figure 2).

4. Procédé selon la revendication 1, caractérisé en ce que au moins les zones en saillie (13) d'un support froncé ou plissé (12) et les éléments (14) en forme de fils ou de bandelettes, qui sont soumis à une tension faible ou nulle et qui sont disposés ou guidés à distance du support (12), sont mis en contact avec le support après avoir été pulvérisés avec l'adhésif fusible (16) ; (figure 16).

5. Procédé selon l'une des revendications 1 à 4, caractérisé en ce que l'adhésif fusible est pulvérisé, sur le ou les éléments (4, 14) en forme de fils ou de bandelettes, sous la forme de filaments pulvérisés en forme de toile d'araignée jusqu'à des filaments pulvérisés se chevauchant en formant un motif et possédant un diamètre de 20 à 400 µm.

6. Procédé selon l'une des revendications 1 à 5, caractérisé en ce que les éléments (4, 14) en forme de fils ou de bandelettes et les zones du support qui se trouvent en dessous sont recouverts d'adhésif pulvérisé sur une largeur (d) de 1 à 15 mm, mesurée à partir du bord longitudinal de chaque élément en forme de fils ou de bandelettes, qui sera relié ultérieurement au support.

7. Procédé selon l'une des revendications 1 à 6, caractérisé en ce que l'adhésif fusible est appliqué sur les éléments (4, 14) en forme de fils ou de bandelettes à raison de 2 à 100 g/m².

8. Procédé selon l'une des revendications 1 à 7, caractérisé en ce que le support (2) utilisé est une feuille en matière synthétique ou un lé de papier ou de non-tissé imprégné.

9. Procédé selon l'une des revendications 1 à 8, caractérisé en ce que, sur le support et sur les éléments en forme de fils ou de bandelettes, on pose, après la pulvérisation de l'adhésif fusible et immédiatement après le guidage conjoint desdits éléments avec le support, une couche de couverture qui est ensuite également comprimée.

10. Procédé selon l'une des revendications 1 à 9, caractérisé en ce qu'on emploie, comme support, une feuille (2) et, comme couche de couverture, un non-tissé (8).

11. Procédé selon l'une des revendications 1 à 9, caractérisé en ce qu'on emploie, comme support, un non-tissé et, comme couche de couverture, une feuille.

12. Procédé selon les revendications 1 à 11, caractérisé en ce qu'il est appliqué pour fabriquer des portions de feuilles froncées formant lés et comportant des éléments extensibles en forme de fils ou de bandelettes dans chaque zone de bord située entre une feuille formant lé et une couche de couverture, une garniture intérieure absorbante disposée entre lesdits éléments et une portion non tissée qui recouvre ladite garniture intérieure, les éléments en forme de fils ou de bandelettes étant, dans ledit procédé, maintenus ou guidés sous une tension d'allongement et étant ensuite pulvérisés par intermittence avec un adhésif fusible,
a) les éléments en forme de fils ou de bandelettes, leurs zones adjacentes et, simultanément, la zone située entre les éléments en forme de fils ou de bandelettes étant pulvérisés avec de l'adhésif fusible, et les éléments en forme de bandelettes étant mis au contact du support par un guidage conjoint, puis
b) un lé en matériau rembourré absorbant étant appliqué sur la zone située entre les éléments en forme de fils ou de bandelettes et pulvérisée avec de l'adhésif fusible,
c) sur la feuille formant lé et sur le lé en matériau rembourré absorbant étant appliqué un lé non-tissé, de façon qu'il couvre le matériau rembourré absorbant et que, en même temps, il se trouve dans la zone des éléments en forme de fils ou de bandelettes pulvérisés avec de l'adhésif fusible,
d) le lé non-tissé étant comprimé avec les éléments en forme de fils ou de bandelettes au moins dans la zone de ceux-ci, et
e) le matériau composé ainsi obtenu étant débité en portions transversalement au sens du lé.

13. Procédé selon la revendication 12, caractérisé en ce que les zones des éléments en forme de fils ou de bandelettes guidés à une distance de 3 à 0,5 cm de la feuille formant lé, lesdites zones étant pulvérisées avec de l'adhésif fusible, sont guidées selon un angle aigu par rapport à la feuille formant lé, après quoi sont appliqués le lé en matériau rembourré absorbant et le lé non-tissé.
